# EUROPEAN PATENT SPECIFICATION

(11) **EP 3 352 737 B1**
(45) Date of publication and mention of the grant of the patent: **31.07.2019**
(21) Application number: 16782311.1
(22) Date of filing: 23.09.2016
(51) Int. Cl.: A61K 9/00, A61K 9/10, A61K 47/44, A61K 31/714

(54) **SUBLINGUAL ADMINISTRATION OF VITAMIN B12 DISPERSED IN A HYDROPHOBIC CONTINUOUS PHASE**
SUBLINGUALE VERABREICHUNG VON IN EINER HYDROPHOBEN KONTINUIERLICHEN PHASE DISPERGIERTEM VITAMIN B12
ADMINISTRATION SUBLINGUALE DE LA VITAMINE B12 DISPERSÉE DANS UNE PHASE CONTINUE HYDROPHOBE

(30) Priority: 25.09.2015 NL 2015506
(43) Date of publication of application: 01.08.2018
(73) Proprietor: Riepma, Klaas Alouis, 6301 GA Valkenburg (NL)
(72) Inventor: Riepma, Klaas Alouis, 6301 GA Valkenburg (NL)
(74) Representative: EP&C
(86) International application number: PCT/NL2016/050659
(87) International publication number: WO 2017/052376

(56) References cited:
- WO-A1-2014/084736
- AMIR SHARABI ET AL: "Replacement therapy for vitamin B12 deficiency: comparison between the sublingual and oral route", BRITISH JOURNAL OF CLINICAL PHARMACOLOGY., vol. 56, no. 6, 14 December 2003 (2003-12-14), pages 635-638, XP055261710, GB ISSN: 0306-5251, DOI: 10.1046/j.1365-2125.2003.01907.x cited in the application

## Description

### Technical field

The present disclosure relates to the field of sublingual administration forms containing vitamin B12 for use in treating a condition in a subject, wherein the condition preferably is vitamin B12 deficiency.

### Background of the disclosure

Vitamin B12 is a vitamin that plays a role in mammalian growth, hematopoiesis, production of epithelial cells and maintainance of the nervous system. It is quite water-soluble and thus could be expected to be easily available to human subjects. However, the absorption from the gut of normal dietary amounts of vitamin B12 is believed to be dependent on gastric Intrinsic Factor (GIF), and the loss of Intrinsic Factor leads to vitamin B12 deficiency. The loss of ability to absorb vitamin B12 (B12) is the most common cause of adult B12 deficiency. Such a loss may, for example, be due to pernicious anemia (with loss of Intrinsic Factor) or to a number of other conditions that decrease production of gastric acid, which also plays a part in absorption of vitamin B12 from foods. Deficiency is most significantly linked to inadequate absorption rather than low consumption, as those who consume high amounts of vitamin B12 may still experience deficiency as evidenced by a low blood concentration.

Vitamin B12 deficiency results in various undesirable conditions such as fatigue, depression, poor memory, etc. Other causes of vitamin B12 deficiency include atrophic gastritis (a thinning of the stomach lining), surgery in which part of the stomach and/or small intestine is removed, conditions affecting the small intestine (such as Crohn's disease, celiac disease, bacterial growth, or a parasite), excessive alcohol consumption, autoimmune disorders (such as Graves' disease or systemic lupus erythematosus) and drug abuse.

Pharmaceutical compositions containing vitamin B12 are known in the art, for example from US5801161 to Merkus, which discloses an intranasal spray. Such a pharmaceutical composition is brightly red, with as the concomitant disadvantage that any fluid of the pharmaceutical composition running from the nose will give the appearance of a bloody nose.

Sharabi et al. (2003 J. Clin Pharmacol., 56, 635-638) disclose tablet formulations that are administered orally or sublingually for correcting vitamin B12 deficiency. However, poor bioavailability of vitamin B12 is demonstrated.

WO2014084736 discloses a pharmaceutical composition comprising vitamin B12 dispersed in a hydrophobic continuous phase which is administered intranasally. The administration form of WO2014084736 also leaves room for improvement.

The object of the present disclosure is to provide an administration form with good bioavailability of vitamin B12, and which is easy and convenient in use.

### Summary of the disclosure

The present disclosure provides vitamin B12 for use in the treatment of a condition in a subject, wherein the vitamin B12 is administered sublingually, and wherein the vitamin B12 is dispersed in a hydrophobic continuous phase. Surprisingly it has been found that oral administration of such a dispersion of solid vitamin B12 (crystalline or amorphic particles) in a hydrophobic continuous phase displays very satisfactory bioavailability.

The vitamin B12 can be comprised in a pharmaceutical composition which e.g. may be in the form of sublingual drops. In the present application, the term vitamin B12 includes cyano-cobalamin, hydroxo-cobalamin, methyl-cobalamin, 5'-deoxyadenosyl-cobalamin, aquacobalamin, glutathionyl-cobalamin and nitrilocobalamin, including the pharmaceutically acceptable salts thereof, and including mixtures thereof. In general, the concentration of vitamin B12 in the hydrophobic continuous phase is between 0.01 - 50 / 75 wt.% with respect to the weight of the hydrophobic continuous phase.

According to a favourable embodiment, the hydrophobic continuous phase is chosen from at least one of i) fat, ii) fatty acids, and iii) wax. Thus, a hydrophobic environment for vitamin B12 is provided. In general, the fatty acids have a length of the carbon chain of at least 6. According to a favourable embodiment, the hydrophobic continuous phase is (edible) oil/fat. The term "hydrophobic" is clear to the skilled person and means that mixing a liquid continuous phase with water forms an emulsion (with or without emulsifier). A hydrophobic phase typically has a water solubility of below 500, 250, 100, 50, 10, preferably below 5, or 1 mg/L pure water at room temperature, i.e. 20 degrees Celcius. If the hydrophobic continuous phase comprises more than one constituent, the average of their water solubility is considered, taking into account their relative wt.% with respect to the hydrophobic continuous phase as a whole.

Such a liquid or fatty pharmaceutical composition is convenient to administer and results in high absorption of vitamin B12 based on concentration in the blood of a human subject.

According to a favourable embodiment, the hydrophobic continuous phase is anhydrous. This promotes the release of vitamin B12 from the pharmaceutically acceptable carrier. Anhydrous, within the context of the present disclosure, means a water content of less than 5 wt.%, preferably less than 1 wt.% and more preferably with less than 0.2 wt.% with respect to the weight of the hydrophobic continuous phase.

According to a favourable embodiment, the hydrophobic continuous phase comprises methylcobalamin or a pharmaceutically acceptable salt thereof as vitamin B12. Methylcobalamin is considered a powerful drug but because it decomposes easily this value has not been realized in oral pharmaceutical compositions according to the prior art as it cannot be stored or pharmaceutical compositions have to be kept frozen. The hydrophobic pharmaceutical composition according to the present disclosure will benefit from improved stability, in particular for methylcobalamin. Without wishing to be bound to any particular theory, it is believed that the fact that vitamin B12 is present as particles reduces its sensitivity to degradation. It is preferred that at least 25 wt.% of vitamin B12 is methylcobalamine or a pharmaceutically acceptable salt thereof, with respect to the weight of the hydrophobic continuous phase.

According to a favourable embodiment, the concentration of vitamin B12 is in the range of 0.05 - 10 wt.% preferably between 0.4 - 8 wt.%, with respect to the hydrophobic continuous phase.

Generally, the amount administered orally will be 25-1500 µl, preferably 50-1000 µl, or 50-500 µl (or 50-2500, or 75-5000 µl).

According to a favourable embodiment, vitamin B12 is colloidally dispersed. Such a pharmaceutical composition is stable for longer periods.

As is clear, the present disclosure relates to vitamin B12 dispersed in a hydrophobic continuous phase, for use in the treatment of a condition in a subject, wherein vitamin B12 is administered sublingually, and wherein the condition for example is vitamin B12 deficiency. Sublingual administration can be seen as the pharmacological route of administration by which the vitamin B12 diffuses into the blood through tissues under the tongue.

It has been found that such a dispersion of solid vitamin B12 particles (crystalline or amorphic) in a hydrophobic continuous phase displays very satisfactory bioavailability. The vitamin B12 deficiency is any condition where an increased level would be of benefit to the subject, which can be a human or an animal. It may be a condition chosen from autism spectrum disorder, fatigue, memory deficiency, ALS, Alzheimer, deficiency caused by drug abuse, thinning of the stomach lining, vitamin B12 deficiency after surgery in which part of the stomach and/or small intestine is removed, Crohn's disease, celiac disease, Graves' disease, systemic lupus erythematosus and migraine.

Finally, the present disclosure relates to vitamin B12 for use in a method of treating a human subject suffering from a condition chosen from vitamin B12 deficiency, autism spectrum disorder, fatigue, memory deficiency, ALS, Alzheimer, deficiency caused by drug abuse, thinning of the stomach lining, vitamin B12 deficiency after surgery in which part of the stomach and/or small intestine is removed, Crohn's disease, celiac disease, Graves' disease, systemic lupus erythematosus and migraine, wherein the pharmaceutical composition according to the present disclosure is administered sublingually.

The method avoids what would appear like a bloody mouth due to the intense red colour of vitamin B12. The disclosure will now be illustrated with reference to the example section below

**Reference Example 1** A composition of 200 mg methyl cobalamin in 10 g coconut fat, and further containing 1 wt.% pepper mint oil was prepared. The composition was administered to a volunteer via the oral route. The volunteer took 1,2 g of the composition (thus containing 20-25 mg Vitamin B12) and allowed it to melt under the tongue for 1 minute, after which the composition was swallowed.

Blood values were measured before and 1 hour after administration.

*Vitamin B12 content before:*
643 pmol/l

*Vitamin B12 content after:*
>1476 pmol/l

The above values demonstrate that Vitamin B12 is absorbed very well when administered orally in a hydrophobic continuous phase. The degree of absorption is surprisingly better when compared to oral administration via tablet formulation. For example, Sharabi et al. (2003 J. Clin Pharmacol., 56, 635-638) demonstrate an increase in Vitamin B12 blood levels of only 10 pmol/l per day for tablet formulations.

### Example 2

Three different products were tested for best vitamin B12 absorption by a volunteer:
(1) a commercial tablet with Hydroxocobalamin (chloride), administered orally;
(2) a liposomal product containing Hydroxococobalamin (chloride) in 10 ml solution build into liposomes (containing water), administered sublingually; and
(3) hydroxocobalamin (chloride) in sesame oil containing the vitamin B12 mixed into sesame oil, administered sublingually (according to the invention).

The sublingual products were held in the mouth for 5 minutes. After these minutes the products were spit out and the mouth rinsed with water. At t = 0 minutes and after 1 hr blood samples were taken and the amount of vitamin B12 measured in the blood.

| Product | Administration route | T = 0 | T = 60 minutes |
|---|---|---|---|
| Hydroxycobalamine10 mg in a tablet | oral | 255 pmol | 268 pmol |
| Hydroxocobalamin 10 mg in a liposomal solution | sublingual | 317 | 365 |
| Hydroxocobalamin 10 mg in sesame oil | sublingual | 412 | 725 |

It can be seen that all products do result in an absorption of Vitamin B12. The best absorption is found with a hydrophobic basis (sesame oil). Further, it shows that the best absorption is achieved when vitamin B12 is administered sublingually in a hydrophobic suspension.

## Claims

1. Vitamin B12 for use in the treatment of a condition in a subject, wherein the vitamin B12 is administered sublingually, and wherein the vitamin B12 is dispersed in a hydrophobic continuous phase.

2. Vitamin B12 for use according to claim 1, wherein the hydrophobic continuous phase is chosen from at least one of i) fat, ii) fatty acids, and iii) wax.

3. Vitamin B12 for use according to any one of the preceding claims, wherein the hydrophobic continuous phase is oil.

4. Vitamin B12 for use according to any one of the preceding claims, wherein the hydrophobic continuous phase is anhydrous.

5. Vitamin B12 for use according to any one of the preceding claims, wherein the vitamin B12 is chosen from at least one of cyano-cobalamin, hydroxo-cobalamin, methyl-cobalamin, 5'-deoxyadenosyl-cobalamin, aquacobalamin, glutathionyl-cobalamin and nitrilocobalamin.

6. Vitamin B12 for use according to any one of the preceding claims, wherein the concentration of vitamin B12 in the hydrophobic continuous phase is in the range of 0.05 - 10 wt.% preferably between 0.4-8 wt.%.

7. Vitamin B12 for use according to any one of the preceding claims, wherein vitamin B12 is colloidally dispersed in the hydrophobic continuous phase.

8. Vitamin B12 for use according to any one of the preceding claims, wherein the subject is a human subject or an animal subject.

9. Vitamin B12 for use according to any one of the preceding claims, wherein the condition is chosen from vitamin B12 deficiency, autism spectrum disorder, fatigue, memory deficiency, ALS, Alzheimer, deficiency caused by drug abuse, thinning of the stomach lining, vitamin B12 deficiency after surgery in which part of the stomach and/or small intestine is removed, Crohn's disease, celiac disease, Graves' disease, systemic lupus erythematosus and migraine.

## Patentansprüche

1. Vitamin B12 zur Verwendung bei der Behandlung einer Erkrankung bei einem Subjekt, wobei das Vitamin B12 sublingual verabreicht wird und wobei das Vitamin B12 in einer hydrophoben kontinuierliche Phase dispergiert ist.

2. Vitamin B12 zur Verwendung nach Anspruch 1,wobei die hydrophobe kontinuierliche Phase ausgewählt ist aus mindestens einem von i) Fett, ii) Fettsäuren und iii) Wachs.

3. Vitamin B12 zur Verwendung nach einem der vorhergehenden Ansprüche, wobei die hydrophobe kontinuierliche Phase Öl ist.

4. Vitamin B12 zur Verwendung nach einem der vorhergehenden Ansprüche, worin die hydrophobe kontinuierliche Phase wasserfrei ist.

5. Vitamin B12 zur Verwendung nach einem der vorhergehenden Ansprüche, wobei das Vitamin B12 ausgewählt ist aus mindestens einem von Cyano-Cobalamin, Hydroxo-Cobalamin, Methyl-Cobalamin, 5'-Deoxyadenosyl-Cobalamin, Aquacobalamin, Glutathionyl-Cobalamin und Nitrilocobalamin.

6. Vitamin B12 zur Verwendung nach einem der vorhergehenden Ansprüche, wobei die Konzentration von Vitamin B12 in der hydrophoben kontinuierlichen Phase im Bereich von 0,05 -10 Gew.-% liegt, vorzugsweise zwischen 0,4 - 8 Gew.-%.

7. Vitamin B12 zur Verwendung nach einem der vorhergehenden Ansprüche, wobei Vitamin B12 in der hydrophoben kontinuierlichen Phase kolloidal dispergiert ist.

8. Vitamin B12 zur Verwendung nach einem der vorhergehenden Ansprüche, wobei das Subjekt ein menschliches Subjekt oder ein tierisches Subjekt ist.

9. Vitamin B12 zur Verwendung nach einem der vorhergehenden Ansprüche, wobei die Erkrankung ausgewählt ist aus Vitamin B12 Mangel, Autismus-Spektrum-Störung, Müdigkeit, Gedächtnisschwäche, ALS, Alzheimer, Mangel durch Drogenmissbrauch, Verdünnung der Magenschleimhaut, Vitamin B12-Mangel nach Operationen, bei denen ein Teil des Magens und/oder des Dünndarms entfernt wird, Morbus Crohn, Zöliakie, Morbus Basedow, systemischer Lupus erythematodes und Migräne.

## Revendications

1. Vitamine B12 destinée à être utilisée dans le traitement d'une affection chez un sujet, dans laquelle la vitamine B12 est administrée par voie sublinguale et dans laquelle la vitamine B12 est dispersée dans une phase continue hydrophobe.

2. Vitamine B12 destinée à être utilisée selon la revendication 1, dans laquelle la phase continue hydrophobe est choisie parmi au moins l'un de i) des corps gras, ii) des acides gras et iii) de la cire.

3. Vitamine B12 destinée à être utilisée selon l'une quelconque des revendications précédentes, dans laquelle la phase continue hydrophobe est de l'huile.

4. Vitamine B12 destinée à être utilisée selon l'une quelconque des revendications précédentes, dans laquelle la phase continue hydrophobe est anhydre.

5. Vitamine B12 destinée à être utilisée selon l'une quelconque des revendications précédentes, dans laquelle la vitamine B12 est choisie parmi au moins l'une de la cyano-cobalamine, l'hydroxo-cobalamine, la méthyl-cobalamine, la 5'-désoxyadénosyl-cobalamine, l'aquacobalamine, la glutathionyl-cobalamine et la nitrilocobalamine.

6. Vitamine B12 destinée à être utilisée selon l'une quelconque des revendications précédentes, dans laquelle la concentration en vitamine B12 dans la phase continue hydrophobe est dans la gamme de 0,05 à 10% en poids, de préférence entre 0,4 et 8% en poids.

7. Vitamine B12 destinée à être utilisée selon l'une quelconque des revendications précédentes, dans laquelle la vitamine B12 est dispersée de manière colloïdale dans la phase continue hydrophobe.

8. Vitamine B12 destinée à être utilisée selon l'une quelconque des revendications précédentes, dans laquelle le sujet est un sujet humain ou un sujet animal.

9. Vitamine B12 destinée à être utilisée selon l'une quelconque des revendications précédentes, dans laquelle l'affection est choisie parmi: une carence en vitamine B12, un trouble du spectre autistique, de la fatigue, un déficit de la mémoire, SLA, la maladie d'Alzheimer, une carence provoquée par un abus de drogues, un amincissement de la muqueuse gastrique, une carence en vitamine B12 après une opération chirurgicale dans laquelle une partie de l'estomac et/ou de l'intestin grêle est prélevée, la maladie de Crohn, une maladie coeliaque, la maladie de Graves, un lupus érythémateux systémique et la migraine.
